(19) Europäisches Patentamt

European Patent Office

Office européen des brevets

(11) EP 0 572 628 B2

(12) **NEW EUROPEAN PATENT SPECIFICATION**

(45) Date of publication and mention
of the opposition decision:
**13.06.2001 Bulletin 2001/24**

(45) Mention of the grant of the patent:
**30.10.1996 Bulletin 1996/44**

(21) Application number: **93901353.8**

(22) Date of filing: **15.12.1992**

(51) Int Cl.$^7$: **C07C 63/38**, C07C 51/493,
C07C 51/09

(86) International application number:
**PCT/US92/10838**

(87) International publication number:
**WO 93/12065 (24.06.1993 Gazette 1993/15)**

(54) **PROCESS FOR PREPARING PURIFIED 2,6-NAPHTHALENEDICARBOXYLIC ACID**

VERFAHREN ZUR HERSTELLUNG VON GEREINIGTEN 2,6-NAPHTHALINDICARBONSÄUREN

PROCEDE DE PREPARATION D'ACIDE 2,6-NAPHTALENEDICARBOXYLIQUE PURIFIE

(84) Designated Contracting States:
**BE CH DE ES FR GB IT LI LU NL**

(30) Priority: **19.12.1991 US 810481**

(43) Date of publication of application:
**08.12.1993 Bulletin 1993/49**

(73) Proprietor: **AMOCO CORPORATION
Chicago, IL 60680-0703 (US)**

(72) Inventors:
• **HOLZHAUER, Juergen, Klaus
Naperville, IL 60540 (US)**
• **ALBERTINS, Rusins
Naperville, IL 60540 (US)**
• **HOOVER, Stephen, Vincent
Aurora, IL 60506 (US)**
• **SIKKENGA, David, Lee
Wheaton, IL 60157 (US)**

(74) Representative: **Schlich, George William et al
Mathys & Squire
European Patent Attorneys,
100 Gray's Inn Road
London WC1X 8AL (GB)**

(56) References cited:
**EP-A- 0 432 910          EP-A- 0 441 347
US-A- 4 302 595**

• **CHEMICAL ABSTRACTS, vol. 102, no. 22, 3 June
1985, Columbus, Ohio, US; abstract no. 185547z,
'PURIFICATION AND IDENTIFICATION OF
2,6-NAPHTALENEDICARBOXYLIC ACID' page 2
;column 1 ; cited in the application**
• **CHEMICAL ABSTRACTS, vol. 83, 1975, abstract
no. 206016g, V. N. KULAKOV et al, "Purification
of 2,6-naphthalenedicarboxylic acid acid", page
369**

EP 0 572 628 B2

## Description

This invention relates generally to an improved process for preparing purified 2,6-naphthalenedicarboxylic acid. More particularly, this invention relates to an improved process for preparing purified 2,6-naphthalenedicarboxylic acid by hydrolyzing a dialkyl-2,6-naphthalenedicarboxylate with water under reaction conditions that provide for large particle size 2,6-naphthalenedicarboxylic acid.

Background of the Invention

Dialkyl-2,6-naphthalenedicarboxylates and 2,6-naphthalene-dicarboxylic acid are useful monomers for the preparation of high performance polymeric materials. For example, dimethyl-2,6-naphthalenedicarboxylate and 2,6-naphthalenedicarboxylic acid can be reacted with ethylene glycol to prepare poly(ethylene-2,6-naphthalate) (PEN). Fibers and film manufactured from PEN have improved strength and superior thermal properties relative to other polyester materials. Films made from PEN demonstrate, for example, superior resistance to gas diffusion and particularly to the diffusion of carbon dioxide, oxygen and water vapor. Because of its exceptional properties, PEN is especially suitable for applications such as food and beverage containers, particularly for so-called "hot-fill" food and beverage containers, tire cord, magnetic recording tape and electronic components.

Although the dialkyl-2,6-naphthalenedicarboxylates -- particularly dimethyl-2,6-naphthalenedicarboxylate -- are suitable monomers for preparing PEN and other polymeric materials, in some commercial-scale operations it is preferable to employ 2,6-naphthalenedicarboxylic acid rather than a dialkyl ester. For example, a polyester manufacturer may have equipment and associated processes available for manufacturing polyesters only from an aromatic dicarboxylic acid. In these circumstances, the diester materials would not be suitable and the use of 2,6-naphthalenedicarboxylic acid would be required. Additionally, it is advantageous to use 2,6-naphthalenedicarboxylic acid in the manufacture of polyesters because the condensation of a diacid with a glycol to form a polyester does not form an alcohol by-product as does the condensation of a diester with a glycol. Polyester manufacturers who use diacids such as 2,6-naphthalenedicarboxylic acid do not, therefore, have to provide for the use or sale of the alcohol by-product.

Methods for preparing 2,6-naphthalenedicarboxylic acid include the bromine-promoted, metal-catalyzed, liquid phase oxidation of 2,6-dialkylnaphthalenes. Such processes are disclosed in U.S. Patent Nos. 3,870,754; 4,950,786 and 4,933,491. The bromine-promoted, metal-catalyzed, liquid phase oxidation of 2,6-dialkylnaphthalenes, particularly 2,6-dimethylnaphthalene, produces a crude product containing a variety of impurities such as brominated 2,6-naphthalenedicarboxylic acids, 2-formyl-6-naphthoic acid, 2-naphthoic acid and trimellitic acid. These impurities, particularly 2-formyl-6-naphthoic acid, are difficult to remove from crude 2,6-naphthalenedicarboxylic acid. The 2,6-naphthalenedicarboxylic acid must, however, be purified before it can be polymerized to form polymeric materials.

The purification of 2,6-naphthalenedicarboxylic acid is considerably more difficult than the purification of a dialkyl-2,6-naphthalenedicarboxylate primarily due to the low solubility of 2,6-naphthalenedicarboxylic acid in most ordinary solvents, and to its high melting point. In the aforementioned U.S. Patent 4,933,491, for example, 2,6-naphthalenedicarboxylic acid was purified only after reacting the 2,6-naphthalenedicarboxylic acid with a lower alkanoic anhydride to produce a component that is soluble in excess alkanoic anhydride. The "solubilized" 2,6-naphthalenedicarboxylic acid was optionally treated with one or more purification procedures. Xu et al. (Chemistry of Synthetic High Polymers, Vol. 10, pp. 107-11, 1984, Chemical Abstracts CA 102: 185547z) describes the purification of 2,6-naphthalenedicarboxylic acid by routine sublimation, recrystallization or distillation as inefficient and difficult due to the poor solubility of 2,6-naphthalenedicarboxylic acid and also because the impurities present, having similar properties, adhere to each other.

In contrast, the diesters of 2,6-naphthalenedicarboxylic acid are considerably more soluble than 2,6-naphthalenedicarboxylic acid in ordinary organic solvents such as xylenes and methanol, and can be purified in the dissolved state. Furthermore, these diesters, particularly the dimethyl ester, are sufficiently volatile so that they can be purified by distillation. Therefore, one potential method for preparing purified 2,6-naphthalenedicarboxylic acid is to convert a purified dialkyl-2,6-naphthalenedicarboxylate to 2,6-naphthalenedicarboxylic acid by reacting the diester with water to hydrolyze the ester bonds and form the free dicarboxylic acid. One such process is disclosed in the aforementioned Xu et al. publication. The process disclosed therein comprises forming purified 2,6-naphthalenedicarboxylic acid by dissolving crude dimethyl-2,6-naphthalenedicarboxylate in a xylene, treating with activated carbon, and then crystallizing purified dimethyl-2,6-naphthalenedicarboxylate. The purified dimethyl-2,6-naphthalenedicarboxylate was subsequently hydrolyzed using a 12% potassium hydroxide solution at reflux conditions, and the solution of hydrolyzed ester was acidified with hydrochloric acid to free purified 2,6-naphthalenedicarboxylic acid. While it is reported that this procedure produces high purity 2,6-naphthalenedicarboxylic acid, the process disclosed would not be desirable for large-scale production. The use of concentrated base to hydrolyze the ester and the required use of an acid to free the salt of 2,6-naphthalenedicarboxylic acid is not economical on a large scale.

Other processes for hydrolyzing dialkyl-2,6-naphthalenedicarboxylates are known. For example, dimethyl-2,6-naphthalenedicarboxylate can be reacted with a molar excess of water in a low temperature, low pressure process, e.g. at 350°-430°F, using an acidic catalyst such as an alkylbenzene sulfonic acid or mineral acid. Such a process produces a 2,6-naphthalenedicarboxylic acid product having a small particle size which is difficult to wash and filter, and that requires large quantities of ethylene glycol to prepare slurries for the manufacture of polyesters such as PEN. Additionally, the hydrolysis reaction is slow under these reaction conditions. In European Patent Application 0432910A, published on June 19, 1991, corresponding to U.S. Patent 5,068,410, a process for hydrolyzing dimethyl-2,6-naphthalenedicarboxylate to 2,6-naphthalenedicarboxylic acid is disclosed wherein an aromatic polycarboxylic acid, for example, pyromellitic acid, trimellitic acid or phthalic acid, is used as a catalyst. It is disclosed therein that these catalysts provide for 2,6-naphthalenedicarboxylic acid with a large particle size, and 2,6-naphthalenedicarboxylic acid having an average particle size as large as 67 microns is described in the examples. It is also taught therein that temperatures in the range of 200-230°C may be used and that at temperatures greater than 230°C the corrosive action of the carboxylic acid is increased and, as a result, corrosion occurs on the surface of the vessel material. The European Patent Application discloses that the concentration of aromatic polycarboxylic acid catalyst may be in the range of 0.2-20% by weight, however, all of the examples in the application utilize an amount of aromatic polycarboxylic acid catalyst that is from 15 to 100 weight percent of the dimethyl-2,6-naphthalenedicarboxylate that is hydrolyzed. In European Patent Application 441347A, a process for hydrolyzing a dialkylester of a naphthalenedicarboxylic acid is disclosed wherein the dialkylester is reacted, in the presence of an esterification catalyst, within a temperature range of 70-350°C and in a solvent inclusive of a monocarboxylic acid containing no unsaturated bond group and having a carbon number of 1-10. It is also disclosed that the solvent contains water in addition to the monocarboxylic acid.

USSR 486,008 [see also Chem. Abstr. 83 (1975), 206016g] describes a method of purifying 2,6-naphthalenedicarboxylic acid, comprising milling unrefined 2,6-naphthalenedicarboxylic acid through a sieve with a 0.05-0.35 mm mesh and then treating the milled product with an aliphatic acid (preferably, acetic acid). The suspension was then cooled and filtered, and the sediment washed with water to yield purified 2,6-naphthalenedicarboxylic acid particle agglomerates, said agglomerates having dimensions of 0.05-0.35 mm.

All of the aforementioned processes for hydrolyzing dimethyl-2,6-naphthalenedicarboxylate require long reaction times and/or the presence of a catalyst or carboxylic acid solvent to carry out the hydrolysis. Additionally, substantially non-porous 2,6-naphthalenedicarboxylic acid having a desirably large particle size of greater than about 100 microns is not disclosed. The art, therefore, needs an improved process for preparing 2,6-naphthalenedicarboxylic acid from a dialkyl-2,6-naphthalenedicarboxylate, and the present invention provides such an improved process. In the present invention, a dialkyl-2,6-naphthalenedicarboxylate is hydrolyzed with water at a temperature of at least about 450°F and the amount of water present is sufficient to solubilize at least about 10% of the 2,6-naphthalenedicarboxylic acid formed. Under these conditions, the hydrolysis of the dialkyl-2,6-naphthalenedicarboxylate is rapid and, particularly when the temperature of the process is about 500°F or greater, the product 2,6-naphthalenedicarboxylic acid is in the form of large substantially non-porous particles having an average size of about 100 microns or greater thereby making the product highly suitable for filtering, washing and preparing PEN. An advantage of the instant invention is that other materials, such as acidic acid catalysts or monocarboxylic acid solvents, need not be added to the hydrolysis mixture and, consequently, need not be separated from the final product as in the prior art processes. Extra processing steps are therefore eliminated.

Additionally, when the process of the instant invention is carried out such that a major portion of the 2,6-naphthalenedicarboxylic acid product is dissolved in the hydrolysis water, the substantially non-porous 2,6-naphthalenedicarboxylic acid produced is in the form of large, well-formed crystals that are superior for forming low viscosity slurries of 2,6-naphthalenedicarboxylic acid in ethylene glycol. These slurries are used for preparing PEN.

Processes for hydrolyzing dimethylterephthalate to terephthalic acid are also known. U.S. Patent 3,594,414 to Katzschmann discloses a process for preparing fiber-grade terephthalic acid comprising hydrolyzing dimethylterephthalate at a temperature of from about 180° to 280°C, preferably 200°-250°C, and preferably in the presence of neutral salts such as sodium chloride, potassium chloride and calcium chloride. In U.S. Patent 4,302,595 to Schoengen et al., a process is disclosed for preparing fiber-grade terephthalic acid from intermediate stage crude dimethylterephthalate wherein in one step of the process the crude dimethylterephthalate, having a limited content of intermediate oxidation products, is hydrolyzed in water in at least two stages at a temperature between 140°C and 350°C preferably from 240° to 280°C in the first stage, and 180° to 220°C in the second stage to produce a reaction mixture containing terephthalic acid. Although a hydrolysis temperature of up to 350°C is disclosed in the Schoengen et al. patent, the examples provided therein use a temperature of 250°C, and it is taught that hydrolysis temperatures above 300°C do not ensure economical operation.

According to the invention there is provided a process for preparing purified 2,6-naphthalenedicarboxylic acid comprising:

a) hydrolyzing, in the absence or substantial absence of at least one of (i) a hydrolysis catalyst, and (ii) a monocarboxylic acid cosolvent, a dialkyl-2,6-naphthalenedicarboxylate with water at a reaction temperature of at least 232.2°C (450°F) under liquid phase conditions and for a time sufficient to convert a major portion of the dialkyl-2,6-naphthalenedicarboxylate to 2,6-naphthalenedicarboxylic acid thereby forming a reaction product mixture, the amount of water present being sufficient to solubilize, at the reaction temperature, at least 10 weight percent of the 2,6-naphthalenedicarboxylic acid formed; and

b) recovering 2,6-naphthalenedicarboxylic acid from the reaction product mixture.

This process produces substantially non-porous 2,6-naphthalenedicarboxylic acid in the form of large particles which are desirable for filtering and washing operations, and for preparing polyesters such as PEN. Additionally, although one or more hydrolysis catalysts can be added to the hydrolysis reaction, the instant process proceeds at high reaction rates and produces large particle size product even in the absence of an added hydrolysis catalyst or cosolvent such as a monocarboxylic acid.

Brief Description of the Figure

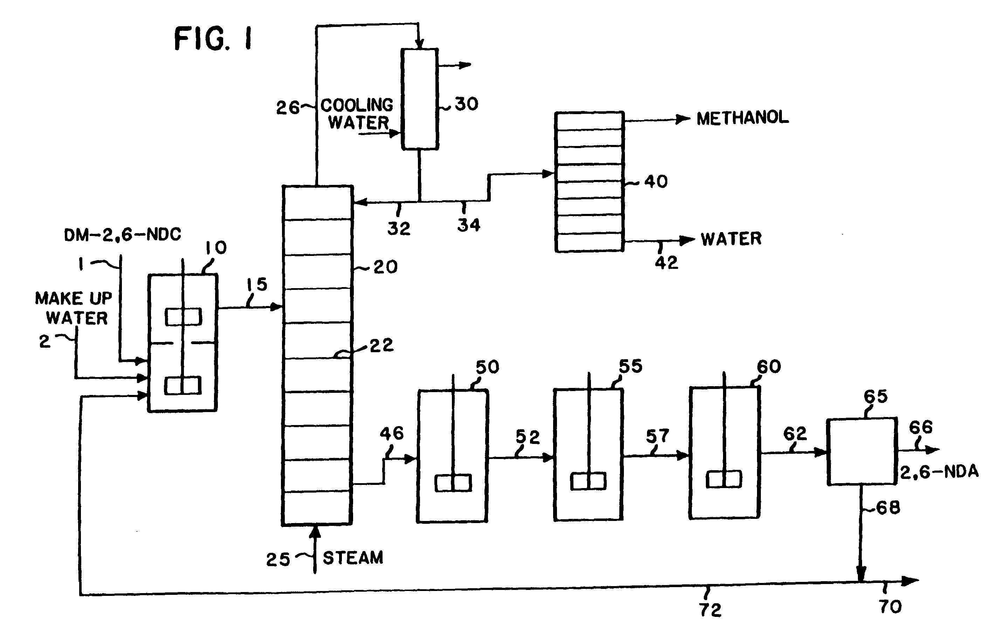

FIGURE 1 is a schematic diagram of a preferred method for operating the process of this invention in the continuous mode.

Detailed Description of the Invention

In the process of the invention a dialkyl-2,6-naphthalenedicarboxylate is reacted with water under liquid phase conditions at an elevated temperature of at least 232.2°C (450°F) to hydrolyze the dialkyl ester and form 2,6-naphthalenedicarboxylic acid. When a pure dialkyl-2,6-naphthalenedicarboxylate is used, purified 2,6-naphthalenedicarboxylic acid is produced. Additionally, the process of this invention can be used to prepare desirably large particle size, substantially non-porous 2,6-naphthalenedicarboxylic acid.

The dialkyl-2,6-naphthalenedicarboxylate hydrolyzed in the process of this invention is preferably a lower dialkyl ester, wherein the alkyl portion of the ester groups contains from 1 to 4 carbon atoms. For example, dimethyl-, diethyl-, di-n-propyl-, diisopropyl, di-n-butyl-, ethylmethyl- and diisobutyl-2,6-napthalenedicarboxylate are suitable dialkyl-2,6-naphthalenedicarboxylates. Dimethyl-2,6-naphthalenedicarboxylate, however, is the most preferred dialkyl-2,6-naphthalenedicarboxylate because it is most easily prepared and purified. The dialkyl-2,6-naphthalenedicarboxylates can be prepared by any known method. For example, they can be prepared by the method disclosed in U.S. Patent No. 4,886,901 to Holzhauer et al. wherein a naphthalenedicarboxylic acid is esterified with methanol and then purified by recrystallization, and they can be prepared by the methods disclosed in U.S. Patent No. 4,847,400 to Steinmetz et al. The dialkyl-2,6-naphthalenedicarboxylates used in the process of this invention can conveniently be prepared by esterifying 2,6-naphthalenedicarboxylic acid prepared by the liquid-phase, cobalt, manganese and bromine catalyzed oxidation of a 2,6-dialkylnaphthalene. Preferably, the 2,6-dialkylnaphthalene is 2,6-dimethylnaphthalene. A suitable method for oxidizing 2,6-dimethylnaphthalene to 2,6-naphthalenedicarboxylic acid is disclosed in Albertins et al., U.S. Patent No. 4,933,491.

Before the dialkyl-2,6-naphthalenedicarboxylate is used in the process of this invention, it is preferably purified to a purity of at least about 95%, preferably at least about 99% and most preferably at least about 99.5% by one or more suitable methods of purification such as recrystallization, distillation, adsorption, sublimation, etc. The combination of recrystallizing dimethyl-2,6-naphthalenedicarboxylate from a solvent such as methanol or xylene, followed by a fractional distillation of the recrystallized dimethyl-2,6-naphthalene-dicarboxylate, is a particularly suitable method for preparing pure dimethyl-2,6-naphthalenedicarbaxylate.

The reaction temperature for the hydrolysis of the dialkyl-2,6-naphthalenedicarboxylate is at least 232.2°C (450°F), preferably at least 260.0°C (500°F) and most preferably at least 298.9°C (570°F). At these reaction temperatures, the hydrolysis reaction proceeds r idly and, as will be described in greater detail hereinbelow, these reaction temperatures provide for the formation of substantially non-porous 2,6-naphthalenedicarboxylic acid having a large particle size making the manipulation of these particles, for example, in washing and filtering procedures easier. A maximum hydrolysis temperature is preferably 371.1°C (700°F).

The pressure for the hydrolysis reaction is a pressure sufficient to maintain a major portion, preferably at least about 75 percent, and more preferably at least about 95 percent of the water in the liquid phase. Suitable reaction pressures are in the range of about 20 atmospheres to about 200 atmospheres.

The amount of water used in the hydrolysis reaction is related to the temperature at which the hydrolysis reaction is conducted. Water should be present in the reaction mixture in an amount sufficient to solubilize, at the reaction temperature, at least about 10 weight percent, preferably at least about 25 weight percent, more preferably at least about 50 weight percent of the 2,6-naphthalenedicarboxylic acid produced in the hydrolysis reaction. Most preferably, the amount of water present in the reaction mixture is an amount sufficient to solubilize all of the 2,6-naphthalenedicarboxylic acid produced in the hydrolysis reaction. We have determined that the hydrolysis reaction proceeds rapidly when the aforementioned amounts of water are present, and, moreover, the 2,6-naphthalenedicarboxylic acid produced under these reaction conditions has an average (mean) particle size of at least about $10^{-4}$ m (100 microns). The amount of water necessary to achieve the hereinabove solubility levels can be determined from the solubility data presented in Example 11. For example, at a reaction temperature of 320°C (608°F), the solubility of 2,6-naphthalenedicarboxylic acid is reported in Example 11 as 33.2 grams per 100 grams of water. Therefore, assuming substantially complete hydrolysis of a sample of dimethyl-2,6-naphthalenedicarboxylate, and if it is desired to operate under reaction conditions where substantially all of the 2,6-naphthalenedicarboxylic acid is solubilized, the weight ratio of water to dimethyl-2,6-naphthalenedicarboxylate charged to the hydrolysis reaction mixture should be at least approximately 2.7:1, respectively. This is because each gram of dimethyl-2,6-naphthalenedicarboxylate produces, after complete hydrolysis, approximately 0.885 gram of 2,6-naphthalenedicarbofylic acid and, based on the solubility data in Example 11, approximately 2.7 grams of water are required to solubilize 0.885 gram of 2,6-naphthalenedicarboxylic acid at 320°C (608°F). This calculation does not take into consideration the amount of water consumed in the reaction; therefore, additional water can be added. Also, this calculation provides the minimum amount of water required to achieve complete solubility. A similar calculation at 260°C (500°F), and assuming 10 percent of the 2,6-naphthalenedicarboxylic acid will be solubilized, results in a value of 3.3 grams of water for each gram of dimethyl-2,6-naphthalenedicarboxylate charged to the hydrolysis reaction. This value also does not take into consideration the consumption of water by the hydrolysis reaction. Therefore, additional water should be added, i.e. at least two moles of water per mole of dialkyl-2,6-naphthalenedicarboxylate charged to the reaction mixture.

These calculations for determining the amount of water charged to the reaction mixture assume that the water contains no other component to affect the solubility of 2,6-naphthalenedicarboxylic acid. Therefore, if other components are added, the solubility data provided in Example 11 may not apply. Also, if the herein disclosed process is practiced wherein water is recycled, the recycled water may contain, depending on the temperature at which the 2,6-naphthalenedicarboxylic acid is partitioned from the water (i.e. mother liquor), various amounts of 2,6-naphthalenedicarboxylic acid. This 2,6-naphthalenedicarboxylic acid should be counted, after adjusting for molecular weight differences, as part of the charge of dialkyl-2,6-naphthalenedicarboxylate for the purpose of determining the level of water charged to the reaction mixture. Therefore, when the water charged to the hydrolysis reaction already contains, for example, dissolved 2,6-naphthalenedicarboxylic acid, the amount of water charged to the hydrolysis reaction should be an amount sufficient to solubilize at least about 10 weight percent, preferably at least about 25 weight percent, more preferably at least about 50 weight percent, and most preferably all of the 2,6-naphthalenedicarboxylic acid in the reaction product mixture.

While the solubility data in Example 11 can be used to determine the amount of water required to achieve the desired dissolution of 2,6-naphthalenedicarboxylic acid in the hydrolysis reaction mixture, it is generally preferable when hydrolyzing the preferred dimethyl-2,6-naphthalenedicarboxylate to use a weight ratio of water to dimethyl-2,6-naphthalenedicarboxylate in the range of at least about 4:1, and preferably at least about 5:1, respectively. The preferred reaction temperature being at least about 260°C (500°F), preferably at least about 282.2°C (540°F), and most preferably at least about 298.9°C (570°F). At these conditions, the hydrolysis rate is rapid and large particle size 2,6-naphthalenedicarboxylic acid is formed. Preferably, the maximum hydrolysis temperature is about 371.1°C (700°F) and, preferably the maximum weight ratio of water to dimethyl-2,6-naphthalenedicarboxylate is about 25:1, more preferably about 10:1, respectively.

It is advantageous to conduct the hydrolysis reaction so that substantially all of the dialkyl-2,6-naphthalenedicarboxylate charged to the reaction mixture is converted to 2,6-naphthalenedicarboxylic acid, thereby eliminating a step to separate the desired 2,6-naphthalenedicarboxylic acid from unreacted diester. However, the hydrolysis reaction can be conducted at lower conversion such as, for example, 50 percent conversion of the dialkyl-2,6-naphthalenedicarboxylate. Therefore, the hydrolysis reaction should be conducted for a time sufficient to convert at least 50 percent of the dialkyl-2,6-naphthalenedicarboxylate, more preferably at least about 95 percent and, as stated above, most preferably for a time sufficient to convert substantially all of the dialkyl-2,6-naphthalenedicarboxylate to 2,6-naphthalenedicarboxylic acid.

When operating the process of this invention under conditions where at least 10 percent and, preferably, where substantially all of the 2,6-naphthalenedicarboxylic acid is solubilized during the reaction, 2,6-naphthalenedicarboxylic acid can be produced having excellent particle size, for example an average particle size of at least about $10^{-4}$ m (100 microns) and preferably at least about $1.25 \times 10^{-4}$ m (125 microns) as measured by a Microtrac™ particle analyzer. Preferably, the average particle size is up to about $10^{-3}$ m (1000 microns), more preferably up to about $8 \times 10^{-4}$ m (800 microns). Furthermore, the 2,6-naphthalenedicarboxylic acid prepared using conditions where substantially all of the acid is solubilized has, in addition, excellent crystal morphology in that the particles are well-defined, individual crystals that are substantially non-porous rather than highly porous aggregates of small crystals. These large, well-formed, well-defined crystals are desirable for forming slurries with, for example, ethylene glycol because the large, well-defined, individual crystals that are substantially non-porous do not require a large quantity of a slurry medium such as ethylene glycol to form an easily mixed and easily pumped slurry of 2,6-naphthalenedicarboxylic acid. More specifically, in a continuous process for preparing PEN, it is desirable to add 2,6-naphthalenedicarboxylic acid to the polymerization reactor as a pumpable slurry of 2,6-naphthalenedicarboxylic acid in ethylene glycol. Furthermore, since the mole ratio of ethylene glycol to 2,6-naphthalenedicarboxylic acid in PEN is 1:1, it is desirable to charge to the polymerization reactor a mole ratio of ethylene glycol to 2,6-naphthalenedicarboxylic acid as close to 1:1 as possible. This is because any excess glycol must be removed in a later stripping step, and excess glycol also leads to the formation of undesirable ethers which can become incorporated into the polyester. The 2,6-naphthalenedicarboxylic acid prepared by the process of this invention wherein a major portion of the 2,6-naphthalenedicarboxylic acid produced in the hydrolysis reaction was solubilized required about 2-3 times less ethylene glycol to achieve the same viscosity of a slurry of ethylene glycol and 2,6-naphthalenedicarboxylic acid wherein the 2,6-naphthalenedicarboxylic acid was prepared by a hydrolysis process conducted at 193.3 - 204.4°C (380-400°F) using p-toluenesulfonic acid as a catalyst and a 5:1 ratio of water to dimethyl-2,6-naphthalenedicarboxylate. At a reaction temperature of 193.3 - 204.4°C (380-400°F) and a weight ratio of water to dimethyl-2,6-naphthalenedicarboxylate of 5:1, only approximately 1.4 weight percent of the 2,6-naphthalenedicarboxylic acid is solubilized. The particles produced by the low-temperature process have a highly porous structure and require larger amounts of ethylene glycol to form a pumpable slurry. Under microscopic examination they appear as an agglomeration of very small crystals. This structure causes the high porosity. European Patent Application WO 90/14375 discloses methods for preparing PEN.

Operating under conditions wherein substantially all of the 2,6-naphthalenedicarboxylic acid produced is solubilized is also advantageous, particularly when using a baffled, plug flow-type reactor or other type of baffled reactor, because plugging problems are eliminated. Whereas, when operating under conditions where a portion of the 2,6-naphthalenedicarboxylic acid is not in solution, the resulting slurry of 2,6-naphthalenedicarboxylic acid in the reactor and piping could lead to plugging.

During the hydrolysis reaction of the dialkyl-2,6-naphthalenedicarboxylate, it is preferable to remove a portion of the alcohol as it is formed. Removal of the alcohol provides for more rapid and more complete conversion of the dialkyl-2,6-naphthalenedicarboxylate to 2,6-naphthalenedicarboxylic acid. The alcohol can be removed from the reaction mixture by, for example, venting a portion of the gaseous phase of the reaction mixture. However, unless provisions are taken to separate the alcohol from the water, this venting procedure will also remove a quantity of water. Depending on the amount of water initially used, this may be a loss of water significant enough to affect the rate of hydrolysis or the dissolution of 2,6-naphthalenedicarboxylic acid. However, any water lost can optionally be replaced. It is advantageous to remove, during the hydrolysis reaction, at least about 30 weight percent of the alcohol, more preferably at least about 90 percent and most preferably at least about 99% of the theoretical amount of alcohol produced by the hydrolysis reaction.

Following the hydrolysis reaction, the reaction mixture is optionally cooled to crystallize the 2,6-naphthalenedicarboxylic acid. When operating under conditions where substantially all of the 2,6-naphthalenedicarboxylic acid is in solution, this cooling step is necessary to recover the 2,6-naphthalenedicarboxylic acid by crystallizing the dissolved 2,6-naphthalenedicarboxylic acid. Preferably, the reaction mixture is cooled to a temperature below about 204.4°C (400°F), more preferably below about 121.1°C (250°F). The rate of cooling affects particle size of the 2,6-naphthalenedicarboxylic acid produced. It is preferable to cool the reaction mixture at a rate that promotes the formation of large particle size 2,6-naphthalenedicarboxylic acid. Cooling rates of less than about 27.8°C (50°F) per minute, preferably less than about 22.2°C (40°F) per minute and most preferably less than about 5.6°C (10°F) per minute provide for large particle size 2,6-naphthalenedicarboxylic acid.

Following the optional cooling step, the 2,6-naphthalenedicarboxylic acid is recovered by partitioning the 2,6-naphthalenedicarboxylic acid from residual water using a suitable means for partitioning a solid phase component from a liquid phase component. For example, solid 2,6-naphthalenedicarboxylic acid can be partitioned from the water phase by filtration, centrifugation, settling, and the like. In this partitioning or separation process the large particle size 2,6-naphthalenedicarboxylic acid produced by the process of this invention is advantageous in that large particles do not "blind" filters and plug centrifuge baskets as readily as fine particles, or retain as much mother liquor, thereby making the partitioning process considerably more efficient. It is desirable to partition the 2,6-naphthalenedicarboxylic acid from the water at an elevated temperature, preferably at a temperature of at least about 65.6°C (150°F), and more preferably at a temperature of at least about 93.3°C (200°F). Filtration at these temperatures provides for a purer 2,6-naphthalenedicarboxylic acid.

After the 2,6-naphthalenedicarboxylic acid is partitioned from the water, it is preferably washed with a suitable solvent such as water, a low molecular weight carboxylic acid, e.g. acetic acid, or an aromatic hydrocarbon such as toluene, xylene, etc. Water is the preferred solvent for washing the 2,6-naphthalenedicarboxylic acid. A suitable amount of solvent to wash the 2,6-naphthalenedicarboxylic acid is an amount such that the weight ratio of solvent to 2,6-naphthalenedicarboxylic acid is at least about 1:1, and preferably at least about 2:1, respectively. It is also preferable to conduct the washing step at an elevated temperature. For example, when water is the solvent for the washing step, it is advantageous for the water to be at a temperature of at least about 65.6°C (150°F). Preferably the water should be at a temperature in the range of about 93.3°C (200°F) to about 300°F. Due to the solubility of 2,6-naphthalenedicarboxylic acid in water, the amount of water used to wash the 2,6-naphthalenedicarboxylic acid preferably should not be an amount that will dissolve more than about 10 weight percent of the 2,6-naphthalenedicarboxylic acid being washed. Otherwise, the losses of 2,6-naphthalenedicarboxylic acid will be too great.

Large particle size and purer 2,6-naphthalenedicarboxylic acid can also be prepared by heating 2,6-naphthalenedicarboxylic acid in water so that the 2,6-naphthalenedicarboxylic acid is partially or, preferably, completely dissolved, followed by slowly cooling the resulting mixture to crystallize 2,6-naphthalenedicarboxylic acid. A suitable temperature for contacting the 2,6-naphthalenedicarboxylic acid with water is a temperature of at least about 232.2°C (450°F), preferably at least about 260°C (500°F), and most preferably at least about 315.6°C (600°F), and preferably up to a temperature of about 371.1°C (700°F). The weight ratio of water to 2,6-naphthalenedicarboxylic acid is preferably at least about 2:1, more preferably at least about 3:1, and most preferably at least about 5:1, respectively. Preferably, the weight ratio of water to 2,6-naphthalene-dicarboxylic acid is no more than about 25:1, more preferably no more than about 10:1, respectively. The mixture of 2,6-naphthalenedicarboxylic acid and water should be maintained at these temperatures for about 0.1 minute to about 6 hours, preferably about 0.1 minute to about 1.0 hour. The rate of cooling the resulting mixture is preferably no greater than about 27.8°C (50°F) per minute, preferably no greater than about 22.2°C (40°F) per minute and most preferably no greater than about 5.6°C (10°F) per minute. Cooling to a temperature of no greater than about 204.4°C (400°F) is suitable. The 2,6-naphthalenedicarboxylate produced is then partitioned from the water.

The hydrolysis process of this invention can be conducted in the batch mode, semi-continuous mode or in the continuous mode. In the batch mode, all of the reactants are charged to a suitable reaction zone at the start of the reaction. As described hereinabove, a portion of the reaction vapor can be released from the reaction zone to further the conversion of the dialkyl-2,6-naphthalenedicarboxylate to 2,6-naphthalenedicarboxylic acid. In the semi-continuous mode of operation, at least one of the reactants is added to the reaction zone during the course of the reaction. For example, a quantity of water can be charged to the reaction zone initially and the diaikyl-2,6-naphthalenedicarboxylate can be added to the reaction zone during the course of the reaction. In the continuous mode of operation, the reactants are added to the reaction zone continuously throughout the course of the reaction and the reaction product mixture is continuously removed from the reaction zone. For commercial-scale operations, it is preferable to operate in the continuous mode. The reaction zone used for a continuous mode of operation can be any suitable reaction apparatus such as at least one continuous stirred tank reactor, a plug flow reactor, a column reactor or a combination of such reactors. The liquid phase reaction residence time, depending on the preselected reaction temperature and ratio of dialkyl-2,6-naphthalenedicarboxylate to water, is suitably about 1 min. to about 5 hrs., preferably about 1 min. to about 2 hours.

A preferred method for conducting the hydrolysis process of this invention in a continuous manner is to use a reactor that is essentially a distillation column equipped with a means for providing liquid hold-up in the column. Suitable means for providing the liquid hold-up include, for example, trays, highly structured packing, and the like. In this method, a dialkyl-2,6-naphthalenedicarboxylate and, optionally, water are introduced near or at the top of the column and an inert gas or, preferably, steam is added to the bottom of the reactor. The addition of steam at the bottom of the reactor is required if water is not added with the dialkyl-2,6-naphthalenedicarboxylate. The steam or inert gas introduced at the bottom of the column reactor provides for the removal of alcohol from the reaction mixture thereby helping to drive the equilibrium controlled reaction to completion. An aqueous solution or slurry of purified 2,6-naphthalenedicarboxylic acid is withdrawn at or near the bottom of the column reactor. This solution or slurry is directed to a crystallization zone, followed by a zone for separating the crystallized 2,6-naphthalenedicarboxylic acid from the water.

Preferably, the vapor exiting the top of the column reactor, which vapor comprises a mixture of alcohol and water, is condensed. Part of the condensate is returned to the top of the column reactor and the remaining part is directed to a means for separating the alcohol from the water, e.g. a distillation column. The feed mixture of dialkyl-2,6-naphthalenedicarboxylate and water (if added) is preferably added to the column reactor at a feed location somewhat below the top of the column. The portion of the column above the feed location will preclude mono-alkyl-2,6-naphthalenedicarboxylate and dialkyl-2,6-naphthalenedicarboxylate from entering and, potentially, fouling the overhead condenser. This column reactor, particularly with the inert gas or steam stripping, provides for a very efficient hydrolysis reaction because alcohol is rapidly separated from the reaction mixture thereby shifting the equilibrium to the desired dicarboxylic acid product.

Before the dialkyl-2,6-naphthalenedicarboxylate is fed to the column reactor, the dialkyl-2,6-naphthalenedicarboxylate can be "prereacted" with water in a prereactor, such as, for example, one or more stirred tank reactors. This prereaction converts the dialkyl-2,6-naphthalenedicarboxylate into a mixture of 2,6-naphthalenedicarboxylic acid and mono-alkyl-2,6-naphthalenedicarboxylic acid. Introducing such a mixture into the column reactor will preclude the presence of a liquid dialkyl-2,6-naphthalenedicarboxylate phase in the column reactor. The melting point of dimethyl-2,6-naphthalenedicarboxylate, for example, is about 374°F (190°C) and, therefore, any dimethyl-2,6-naphthalene that is present in the reactor will exist as a separate liquid phase. The presence of two liquid phases in the column reactor is not desirable because the contents of the column reactor are not mechanically agitated, although such agitation could be provided, if required, by a suitable agitating means. A suitable liquid phase residence time in the column reactor is about 1 min. to about 5 hours. The reaction temperature and water levels in the column reactor are the same as those described hereinabove for the hydrolysis reaction. In order to preclude plugging the column reactor, it is most preferable to operate the reaction under conditions wherein all or substantially all of the 2,6-naphthalenedicarboxylic acid is in solution. Additionally, complete solubilization provides for the largest 2,6-naphthalenedicarboxylic acid particles and 2,6-naphthalenedicarboxylic acid in the form of well-formed, substantially individual crystals having substantially no internal porosity.

Figure 1 represents, in schematic form, the preferred method for conducting the continuous hydrolysis of a dialkyl-2,6-naphthalenedicarboxylate to form purified 2,6-naphthalenedicarboxylic acid. For the purposes of this description, dimethyl-2,6-naphthalenedicarboxylate is the diester that is hydrolyzed. The reaction temperatures and quantity of water in the prereactor and column reactor, as well as the crystallization and filtration temperatures, are as described hereinabove.

Referring to Figure 1, dimethyl-2,6-naphthalenedicarboxylate, preferably in molten form, is added to prereactor 10 along with water using feed lines 1 and 2, respectively. The liquid-phase residence time of the reaction mixture in reactor 10 is preferably sufficient to convert most of the dimethyl-2,6-naphthalenedicarboxylate to a mixture of 2,6-naphthalenedicarboxylic acid and mono-methyl-2,6-naphthalenedicarboxylic acid. Prereactor 10 comprises a stirred tank reactor having a lower and upper section separated by a baffle. The reaction mixture exits from the upper section of prereactor 10 through line 15 and is directed to the upper part of column reactor 20. Column reactor 20 comprises a vertical column equipped with a plurality of trays 22 to provide liquid holdup in the column. Steam is added to the bottom of column 20 through steam line 25. Overhead vapor is removed from column reactor 20 and is directed through line 26 to condenser 30. Condensate from condenser 30 is partitioned and part of the condensate is returned to column 20 through line 32 and part is directed through line 34 to distillation column 40 where methanol is separated from water. The water is recycled to prereactor 10 through line 42 and the methanol is directed to, for example, an esterification reactor (not shown) for converting 2,6-naphthalenedicarboxylic acid to dimethyl-2,6-naphthalenedicarboxylate. The hydrolysis reaction product, preferably having all the 2,6-naphthalenedicarboxylic acid dissolved in the reaction mixture, exits column reactor 20 through line 46 located near the bottom of column. The reaction mixture passes through a series of stirred tank vessels 50, 55 and 60 where the reaction mixture is cooled to slowly crystallize the 2,6-naphthalenedicarboxylic acid. Cooling can be accomplished by lowering the pressure and allowing the reaction mixture to cool by evaporative cooling. Vessels 50, 55 and 60 can be substituted with one or more batch crystallizers or with a substantially plug flow-type crystallizer. Product slurry exits last crystallizer 60 through line 62 and is directed to centrifuge 65 wherein the product 2,6-naphthalenedicarboxylic acid is separated from mother liquor. Product exits the centrifuge through line 66 and is typically sent to a dryer (not shown). Mother liquor is removed from centrifuge through line 68 and is recycled through line 72 to prereactor 10. Part of the mother liquor is purged through line 70.

An advantage of the present invention is that the hydrolysis reaction is rapid and large particle size 2,6-naphthalenedicarboxylic acid is produced without the inclusion of a hydrolysis catalyst or other component such as a monocarboxylic acid co-solvent in the reaction mixture. Consequently, this invention is the hereinabove described hydrolysis reaction carried out in the substantial absence and, preferably, in the absence of a hydrolysis catalyst and/or monocarboxylic acid co-solvent. However, if desired, a hydrolysis catalyst can be added to the hydrolysis reaction mixture. For example, from about 0.001 to about 2.0 weight percent of a catalyst, based on the weight of the dialkyl-2,6-naphthalenedicarboxylate charged to the hydrolysis reaction, can be used. Suitable catalysts include, for example, strong acids such as hydrochloric acid and sulfuric acid; alkyl or aryl sulfonic acids, such as toluene sulfonic acid; or one or more metal-based catalysts, such as, for example, an oxide, halide, sulfate or carboxylic acid salt of antimony, copper, zinc, etc.

This invention is also 2,6-naphthalenedicarboxylic acid produced by the hydrolysis process described herein, particularly when the hydrolysis reaction is conducted at temperatures of at least about 260°C (500°F), and more preferably at temperatures of at least about 282.2°C (540°F), and most preferably at temperatures of at least about 298.9°C (570°F). The 2,6-naphthalenedicarboxylic acid produced by the process of this invention is characterized by its large particle size wherein the average particle size, as measured by a Microtrac™ particle size analyzer, is at least about $10^{-4}$ m (100 microns) and preferably at least about $1.25 \times 10^{-4}$ m (125 microns). Preferably, the average particle size is up to about $10^{-3}$ m (1000 microns), more preferably up to about $8 \times 10^{-4}$ m (800 microns). Additionally, this invention includes the compositions comprising a physical mixture of 2,6-naphthalenedicarboxylic acid having an average particle size of at least about $10^{-4}$ m (100 microns), preferably at least about $1.25 \times 10^{-4}$ m (125 microns), and preferably up to about $10^{-3}$ m (1000 microns), more preferably up to about 800 microns, and a glycol containing 2 to 6 carbon atoms, and preferably ethylene glycol, wherein the mole ratio of glycol to 2,6-naphthalenedicarboxylic acid is about 1:1 to about 10:1, preferably 1:1 to about 4:1, respectively.

This invention is also 2,6-naphthalenedicarboxylic acid having an average particle size of at least about 100 microns, preferably at least about 125 microns, and preferably up to about 1000 microns, more preferably up to about $8 \times 10^{-4}$ m (800 microns), wherein the 2,6-naphthalenedicarboxylic acid is in the form of well-formed crystals having substantially no internal porosity and which, therefore, do not require large amounts of ethylene glycol or other glycol such as 1,4-dihydroxybutane to form a pumpable slurry; and this invention includes physical mixtures of such 2,6-naphthalenedicarboxylic acid with a glycol containing 2 to 6 carbon atoms, preferably ethylene glycol, wherein the mole ratio of glycol to 2,6-naphthlenedicarboxylic acid is about 1:1 to about 10:1, and preferably 1:1 to about 4:1, respectively.

Furthermore, this invention is a composition consisting essentially of a physical mixture of 2,6-naphthalenedicarboxylic acid and a glycol wherein the composition has a Brookfield viscosity of no more than about 1 Pa s (1000 centipoise) measured using a number 4 spindle rotated at 50 rpm, and wherein the molar ratio of glycol to 2,6-naphthalenedicarboxylic acid is about 1:1 to about 4:1, preferably about 2:1 to about 4:1, respectively. The glycol contains 2 to 6 carbon atoms, most preferably the glycol is ethylene glycol. This invention is also a composition consisting essentially of a physical mixture of 2,6-naphthalenedicarboxylic acid and a glycol wherein the Brookfield viscosity of the composition (number 4 spindle, 50 rpm) is no more than about 3 Pa s (3000 centipoise) and the mole ratio of glycol, preferably ethylene glycol, to 2,6-naphthalenedicarboxylic acid is about 1:1 to about 3.5:1, preferably about 2:1 to about 3.5:1, respectively.

The following examples demonstrate the hydrolysis of dimethyl-2,6-naphthalenedicarboxylate according to the process of this invention. These examples also demonstrate the hereinabove described compositions. In these examples, dimethyl-2,6-naphthalenedicarboxylate is referred to as DM-2,6-NDC, the mono-methyl ester of 2,6-naphthalenedicarboxylic acid is MM-2,6-NDC, 2,6-naphthalenedicarboxylic acid is 2,6-NDA, 2-naphthoic acid is 2-NA, and dimethyl ether is DME. The DM-2,6-NDC used for these examples was obtained by esterifying the 2,6-NDA formed by the liquid phase oxidation of 2,6-dimethylnaphthalene using cobalt, manganese and bromine as the oxidation catalyst. The DM-2,6-NDC was esterified and purified according to the procedure disclosed in U.S. Patent 4,886,901 to Holzhauer et al. The organic components were analyzed by liquid chromatography, the metal analyses were conducted by Inductively Coupled Plasma (ICP) analysis. ND means not detected.

Color of the 2,6-naphthalenedicarboxylate was evaluated visually as noted, and it was evaluated by its optical density (OD) at 380 nm. Optical density at 380 nm is the absorbance of a 0.5% (weight/volume) solution in a 50 mm pathlength cell. A Perkin-Elmer 552A UV-Vis spectrophotometer (or similar instrument) can be used. In this procedure, after calibrating the spectrophotometer at 380 nm with the reference and sample cells containing 4.0 N $NH_4OH$, the sample cell of the spectrophotometer is filled with a solution prepared by dissolving 0.25 grams of 2,6-naphthalenedicarboxylic acid in 50.0 ml of 4.0N $NH_4OH$. The absorbance at 380 nm (read within one hour of sample preparation) is the OD at 380 nm.

The color of the 2,6-naphthalenedicarboxylic acid was also evaluated by Tri-stimulus Color measurements, L, a* and b*. The measurement of the b*-value of a solid on the Hunter Color Scale is described in Hunter, The Measurement of Appearance, Chapter 8, pp. 102-132, John Wiley & Sons, N.Y., N.Y. (1975), and in Wyszecki et al., Color Science. Concepts and Methods, Quantitative Data and Formulae, 2d Ed., pp. 166-168, John Wiley & Sons, N.Y., N.Y. (1982).

More specifically, the b*-value of purified 2,6-naphthalenedicarboxylic acid was determined using a Diano Match Scan Spectrophotometer as follows. 2,6-Naphthalenedicarboxylic acid was pressed into a pellet by placing 7 grams of 2,6-naphthalenedicarboxylic acid into a 32 mm mold and applying 48.3 MPa (7000 psi) pressure for at least 90 seconds. The pellet was then irradiated with white light that was UV-filtered. The spectrum of the visible light reflected from the sample was determined and Tri-stimulus values (X, Y, and Z) were computed using the CIE Standard Observer functions. Using the weighted-ordinate method, Tri-stimulus values are obtained from the following equations:

$$X = \sum_{400}^{700} R_\lambda \bar{x}_\lambda , \quad Y = \sum_{400}^{700} R_\lambda \bar{y}_\lambda , \quad Z = \sum_{400}^{700} R_\lambda \bar{z}_\lambda ,$$

where $R_\lambda$ is the percent reflectance of the object at wavelength $\lambda$ and $\bar{x}_\lambda$, and $\bar{y}_\lambda$, and $\bar{z}_\lambda$ are the Standard Observer functions at wavelength $\lambda$ CIE Illuminant D65. The Tri-stimulus values, X, Y and Z, identify the color of the object in terms of the mixture of the primary lights that match it visually. Tri-stimulus values, however, are of limited use as color specifications because they do not correlate with visually meaningful attributes of color appearance and are not uniform in the spacing of colors as related to visual differences. As a result, "Unfiorm Color Scales" (UCS) have been adopted which use simple equations to approximate visual response. The UCS scale used by the Diano instrument is the CIE 1976 $L^*a^*b^*$ formula which converts Tri-stimulus values to $L^*$, $a^*$, and $b^*$ values as shown below:

$$L^* = 25(100Y/Y_o)^{1/3} - 16$$

$$a^* = 500[(X/X_o)^{1/3} - (Y/Y_o)^{1/3}]$$

$$b^* = 200[(Y/Y_o)^{1/3} - (Z/Z_o)^{1/3}]$$

The L*-value is a measure of the luminosity or whiteness of an object where L* = 100 is pure white, L* = 0 is black, and in between is gray. The L*-value is strictly a function of the Tri-stimulus Y-value. The b*-value is a measure of the yellowness-blueness attribute where positive b*-values represent yellow appearance and negative b*-values represent blue appearance. The b*-value is a function of both Tri-stimulus values Y and Z.

Particle size was measured using a Microtrac II™ Standard Range Analyzer manufactured by Leeds and Northrup Co., St. Petersburg, Florida. Methanol (or water) was used as circulating liquid for suspending the 2,6-naphthalenedicarboxylic acid particles. This method is a based on laser light scattering, and provides both a mean (average) and median value for the particles measured.

It is to be understood that the following examples are being provided to set forth embodiments of the present invention and are not intended to limit the scope thereof.

Example 1 - Comparative

Dimethyl-2,6-naphthalenedicarboxylate was hydrolyzed in the batch mode using a 3.33:1 weight ratio of distilled water to dimethyl-2,6-naphthalenedicarboxylate using p-toluenesulfonic acid as a catalyst. A stirred, 6-gallon pressure vessel constructed of 316 stainless steel was used for the hydrolysis. The reaction times, pressure, temperature and analysis of the resulting 2,6-naphthalenedicarboxylic acid are provided in Table 1. In each of Runs 1 and 2, as noted, the product from an initial four hour reaction was reacted a second time under identical conditions for an additional four hours using fresh water and a fresh catalyst charge. 1950 Grams of dimethy-2,6-naphthalenedicarboxylate were charged to the reactor. After filtration at room temperature, the 2,6-NDA product was washed with about 1200 grams of water.

The solubility of 2,6-naphthalenedicarboxylic acid in water at 430°F (220°C) is approximately 0.5/100 g (See Example 11). Therefore, at these reaction conditions, assuming 100% conversion to 2,6-naphthalenedicarboxylic acid, it is calculated that only about 1.9 weight percent of the 2,6-naphthalenedicarboxylic acid product was in solution at the end of the hydrolysis reaction. As the data in Table 1 show, the 2,6-naphthalenedicarboxylic acid produced had a median particle size of about 6.8 x 10$^{-6}$ m (6.8 microns).

TABLE 1

| | Run 1 | Run 2 |
|---|---|---|
| Solvent Ratio[a] | 3.33:1 | 3.33:1 |
| Wt.% p-TSA[b] | 0.78 | 0.78 |
| Reaction Temp., °F (°C) | 430 (220) | 430 (220) |
| Reaction Press., psig (MPa) | 350 (2.5) | 350 (2.5) |
| Reaction Time, hrs | 4+4 | 4+4 |
| Product Analysis | | |
| Visual Color | White | White |
| OD (at 380 nm) | 0.025 | 0.035 |
| Median Particle Size*, (Microns) | 6.8 | 6.7 |
| Mean Particle Size (Microns) | 11.5 | 6.8 |
| Wt.% DM-2,6-NDC | ND | 0.04 |
| Wt.% MM-2,6-NDC | 0.17 | 0.21 |
| Metal Analysis[c] ppm | | |
| Al | 2.0 | 1.9 |
| Cr | 1.6 | 1.6 |
| Fe | 3.1 | 3.0 |

[a] Weight ratio of water to DM-2,6-NDC.

[b] Weight percent of p-toluene sulfonic acid based on DM-2,6-NDC.

[c] Only for metals at greater than 1 ppm.

* $10^{-6}$ m

Example 2

Dimethyl-2,6-naphthalenedicarboxylate was hydrolyzed at elevated temperatures and elevated pressures in the batch-mode in a stirred, 300 ml Hastelloy C autoclave reactor. 37.5 Grams of dimethyl-2,6-naphthalenedicarboxylate were charged to the reactor. No catalyst was used. The 2,6-NDA product was separated from the mother liquor by filtration at room temperature followed by washing with about 150 grams of water. The reaction time, temperature, pressure and analysis of the resulting 2,6-naphthalenedicarboxylic acid are provided in Table 2. During each of the runs reported in Table 2, approximately one-half of the solvent was unintentionally lost. This loss of solvent likely caused the removal of methanol from the reaction mixture thereby shifting the equilibrium towards 2,6-naphthalenedicarboxylic acid. Runs 1, 2 and 3 reported in Table 2 were run consecutively in the same autoclave reactor.

These data demonstrate that the hydrolysis reaction is rapid at temperatures of 282.2°C (540°F) and greater in the absence of a catalyst. A product containing only about 0.144 wt.% MM-2,6-NDC was obtained which result is superior to that using the two-stage reaction reported in Table 1. Additionally, the particle size of the 2,6-naphthalenedicarboxylic acid produced by this process is approximately 4 to 7 times larger than the 2,6-naphthalenedicarboxylic acid produced by the low-temperature hydrolysis reported in Table 1.

The metals in the product 2,6-naphthalenedicarboxylic acid are due either to corrosion of the Hastelloy C autoclave or, more likely, to contaminants from previous uses of the autoclave reactor. However, as shown by Example 6, Run 2, which used a much larger reactor and, consequently, a much smaller ratio of reactor surface to reaction mixture, metal contamination was not observed to a significant extent in the product. The color data reported in Table 2 show that the product is gray-colored, however, the OD measurements, which are measured using a filtered solution of the 2,6-NDA in aqueous base, are quite low at 0.015-0.025, indicating that the color-causing impurities are most likely inorganic contaminants.

Based on the data from Example 11, the solubility of 2,6-naphthalenedicarboxylic acid in water at 282.2°C (540°F) is approximately 6.6 g/100 g. Therefore, at the reaction conditions shown for Run 2, assuming 100% conversion to 2,6-naphthalenedicarboxylic acid, it is calculated that 30 weight percent of the 2,6-naphthalenedicarboxylic acid product was solubilized. At 310°C (590°F), the solubility of 2,6-naphthalenedicarboxylic acid in water is 22.7g/100 g.

Therefore, the reaction conditions for Runs 1 and 3, assuming approximately 100% conversion to 2,6-naphthalenedicarboxylic acid, it is calculated that approximately all of the product acid was solubilized throughout the hydrolysis reaction. Since it was not possible to tell when the solvent was lost during these runs, the effect of the loss of solvent on the particle size cannot be determined. Nevertheless, exceptionally large particle size 2,6-naphthalenedicarboxylic acid was formed by this process as shown by the particle size data.

TABLE 2

|  | Run 1 | Run 2 | Run 3 |
|---|---|---|---|
| Solvent Ratio[a] | 4:1 | 4:1 | 4:1 |
| Catalyst | None | None | None |
| Reaction Temp., °F/°C | 590/310 | 540/282.2 | 590/310 |
| Reaction Press., psig/MPa | 1370/9.6 | 920/6.4 | 1530./10.6 |
| Reaction Time, hrs. | 1.0 | 1.0 | 0.17 |
| Product Analysis |  |  |  |
| Visual Color | Gray | Gray | Gray |
| OD (380 nm) | 0.020 | 0.015 | 0.025 |
| Median Particle Size x $10^{-6}$ m (Microns) | 30.1 | 116.9 | 110.3 |
| Mean Particle Size x $10^{-6}$ m (Microns) | 34.4 | 142.9 | 130.7 |
| Wt.% DM-2,6-NDC | <0.032 | 0.061 | 0.446 |
| Wt.% MM-2,6-NDC | 0.144 | 1.20 | 6.76 |
| Metal Analysis,[b] ppm |  |  |  |
| Al | 8.9 | 6.0 | 4.8 |
| Ca | 2.3 | 3.6 | 2.0 |
| Co | 21.9 | 13.6 | 1.31 |
| Cr | 39.5 | 19.9 | 11.4 |
| Cu |  | 1.9 |  |
| Fe | 123 | 48 | 7.3 |
| K | 1.8 |  | 2 |
| Mg |  | 12.6 | 4.3 |
| Mn | 1.02 | 3.19 | 1.3 |
| Mo Na | 21.2 | 15.9 1.7 | 15.7 |
| Ni | 83 | 25.3 | 7.6 |
| Ti | 1.53 |  |  |
| Zn |  | 1.8 |  |

[a] Weight ratio of water to DM-2,6-NDC

[b] Only for metals at greater than 1 ppm.

Example 3

Dimethyl-2,6-naphthalenedicarboxylate was hydrolyzed in the batch-mode using a 1 liter titanium pressure reactor in the absence of a catalyst. 115.5 Grams of dimethyl-2,6-naphthalenedicarboxylate were charged to the reactor. Product was separate from mother liquor by filtration at room temperature followed by washing with about 450 grams of water. The reaction time, reaction temperature, reaction pressure and analysis of the resulting 2,6-naphthalenedicarboxylic acid are provided in Table 3. Runs 1-3 in Table 1 were run consecutively in the same titanium reactor.

These data show the rapid hydrolysis of dimethyl-2,6-naphthalenedicarboxylate at about 282.2°C (540°F) and 310°C (590°F) in the absence of a catalyst. Additionally, these data show that the methanol recovery was less than quantitative, possibly due to the formation of dimethylether. As in the prior example, large particle size, 2,6-naphthalenedicarboxylic acid was produced, i.e. a median particle size of 188.3 microns.

12

Example 4

Table 4 lists the results of the hydrolysis of dimethyl-2,6-naphthalenedicarboxylate in the batch-mode using a stirred, 2 liter, 316 stainless steel pressure reactor. No hydrolysis catalyst was used. The reactor was charged with 200 grams of dimethyl-2,6-naphthalenedicarboxylate and 1000 grams of distilled water. In each of Runs 1-3, the product 2,6-naphthalenedicarboxylic acid was separated from the remaining water, dried and analyzed.

During Runs 2 and 3, a portion of the gas phase of the reaction mixture was vented to remove water and methanol hydrolysis product. For Run 2,110 grams of a gaseous mixture of methanol and water were removed from the reaction 15 minutes after the reaction mixture reached the noted reaction temperature. In Run 3, the reactor was vented twice, once as soon as the reactor reached the noted reaction temperature, and a second time 20 minutes later. In the first venting, 99 grams of a gaseous mixture of water and methanol were removed. In the second venting, 155 grams were removed. For Run 4, the reaction mixture was vented three times. Once when the reaction mixture reached the reaction temperature, then two more times at 10 minute intervals.

The solid reaction product comprising mainly 2,6-naphthalenedicarboxylic acid was washed, as noted, by slurrying the product with three parts by weight of water for 30 minutes followed by filtration.

These data demonstrate that the hydrolysis reaction is rapid at 315.6°C (600°F) and that the venting of the reactor reduces the amount of MM-2,6-NDC and DM-2,6-NDC in the product.

TABLE 3

|  | Run 1 | Run 2 | Run 3 |
|---|---|---|---|
| Solvent Ratio[a] | 4:1 | 4:1 | 4:1 |
| Catalyst | None | None | None |
| Reaction Temp., °F/°C | 587/308.3 | 588/308.9 | 537/280.6 |
| Reaction Press., psig/MPa | 1410/9.8 | 1420/9.9 | 970/6.8 |
| Reaction Time, hrs. | 1.0 | 0.25 | 1.0 |
| Product Analysis |  |  |  |
| Visual Color | Gray | Off-White | Off-White |
| OD (380 nm) | 0.05 | 0.01 | 0.05 |
| Median Particle Size x $10^{-6}$ m (Microns) | 188.3 | - | - |
| Mean Particle Size x $10^{-6}$ m (Microns) | 214.8 |  |  |
| Wt.% DM-2,6-NDC | 0.29 | 0.496 | 0.329 |
| Wt.% MM-2,6-NDC | 3.75 | 4.89 | 2.95 |
| Recovered Methanol[b] | 51.6 | 74.7 | 86.8 |
| Metal Analysis,[c] ppm |  |  |  |
| Al | 5.9 | 3.19 | 10.3 |
| Ca | 3.7 |  | 6.5 |
| Fe | 2.27 | 2.79 | 2.27 |
| K |  | 1.8 | 2.1 |
| Mg |  |  | 1.79 |
| Ni | 1.2 |  | 1.46 |
| Ti | 7.6 | 14.6 | 16.6 |
| Zr |  | 3.49 | 4.1 |

[a] Weight ratio of water to DM-2,6-NDC

[b] % of theoretical

[c] Only for metals greater than 1 ppm

TABLE 4

| | Run 1 | Run 2 | | Run 3 | | Run 4 |
|---|---|---|---|---|---|---|
| Solvent Ratio[a] | 5:1 | 10:1 | | 10:1 | | 10:1 |
| Catalyst | None | None | | None | | None |
| Reaction Temp., °F/°C | 600/315.6 | 600/315.6 | | 600/315.6 | | 600/315.6 |
| Reaction Time,[b]hrs. | 0.25 | 0.5 | | 0.5 | | 0.5 |
| Times vented | 0 | 1 | | 2 | | 3 |
| Filter Cake Washed | <u>No</u> | <u>No</u> | <u>Yes</u> | <u>No</u> | <u>Yes</u> | <u>No</u> |
| Product Analysis | | | | | | |
| Wt.% DM-2,6-NDC | 0.28 | 0.10 | 0.16 | 0.09 | 0.04 | 0.04 |
| Wt.% MM-2,6-NDC | 2.9 | 1.4 | 1.4 | 0.63 | 0.71 | 0.86 |
| Wt.% 2-NA | 0.19 | 0.34 | 0.18 | 0.05 | 0.05 | 0.41 |
| Visual Color | cream | - | - | - | - | beige |

[a] Weight ratio of water to DM-2,6-NDC

[b] Time at 600°F/315.6°C

Example 5

The hydrolysis of dimethyl-2,6-naphthalenedicarboxylate was conducted in the batch-mode in a 2 liter, stirred, titanium-lined reactor. The reaction time, temperature, pressure and analysis of the resulting 2,6-naphthalenedicarboxylic acid are provided in Table 5.

In Run 1, the reactor was charged with 150 grams of dimethyl-2,6-naphthalenedicarboxylate and 1500 grams of distilled water. When the reaction mixture reached 315.6°C (600°F), 75 grams of the gaseous mixture of water and methanol were vented from the reactor. This venting was repeated 20 minutes later. After a total reaction time of 30 minutes, the reaction mixture was cooled and, at a temperature of 153.9°C (309°F), the mother liquor was removed through a filter located at the bottom of the reactor. At this point, while the reactor was still at about 153.9°C (309°F), 300 grams of fresh distilled water were added to the reactor and the resulting slurry was stirred for about 15 minutes. The slurry was again filtered and the 2,6-naphthalenedicarboxylic acid product was dried and analyzed.

Run 2 was conducted in substantially the same manner except that 175 grams of dimethyl-2,6-naphthalenedicarboxylate were used, approximately 150 grams of the gaseous mixture of methanol and water were vented during each venting, the mother liquor was separated from the 2,6-naphthalenedicarboxylic acid product at 166.1°C (331°F), and the product was washed using 500 grams of distilled water at a temperature of about 121.1°C (250°F).

These data demonstrate that product of good color and low levels of DM-2,6-NDC and MM-2,6-NDC can be obtained using the process of this invention. The hot filtration appears to have reduced the level of 2-NA in the product relative to the results reportecl in Table 4. Additionally, the product filtered at the higher temperature exhibited superior color.

TABLE 5

| | Run 1 | Run 2 |
|---|---|---|
| Solvent Ratio[a] | 10:1 | 10:1 |
| Catalyst | None | None |
| Reaction Temp., °F/°C | 600/315.6 | 600/315.6 |

[a] Weight ratio of water to DM-2,6-NDC

TABLE 5 (continued)

|  | Run 1 | Run 2 |
|---|---|---|
| Reaction Time,[b] hrs. | 0.5 | 0.5 |
| Times Vented | 2 | 2 |
| Filtration Temperature; °F/°C | 309/153.9 | 333/166.1 |
| Product Analysis |  |  |
| Wt.% DM-2,6-NDC | 0.06 | 0.09 |
| Wt.% MM-2,6-NDC | 0.07 | 0.23 |
| Wt.% 2-NA | Not detected | Not detected |
| Visual Color | Off-white | White |
| Tri-stimulus Color |  |  |
| L | 91.27 | 94.85 |
| $a^*$ | -0.03 | -0.71 |
| $b^*$ | 4.97 | 3.7 |

[b] Time at 600°F/315.6°C

Example 6

Dimethyl-2,6-naphthalenedicarboxylate was hydrolyzed in a 25 gallon, 316 stainless steel reactor in two separate runs according to the following procedure:

In Run 1, the reactor was charged with 23.9 Ibs of dimethyl-2,6-naphthalenedicarboxylate and 110 Ibs of distilled water. After 0.5 hours at the reaction temperature, the reactor was cooled to 125.6°C (258°F) and the product 2,6-naphthalenedicarboxylic acid was filtered from the water. The filter cake was washed with 11.3 kg (25 Ibs) of near-boiling distilled water, and the product was dried and analyzed.

In Run 2, the reactor was charged with 11.3 kg (25 Ibs) of dimethyl-2,6-naphthalenedicarboxylate and 49.9 kg (110 Ibs) of water. The reaction mixture was maintained at the reaction temperature for 20 minutes and then cooled to 129.4°C (265°F) for filtration. The filter cake was washed with 22.7 kg (50 Ibs) of hot distilled water before it was dried and analyzed.

Except for MM-2,6-NDC and DM-2,6-NDC, the products from these reactions contained 0.03 are 0.043 weight percent 2-NA, respectively, as the only significant impurity. Additionally, metals analysis of the product from Run 2 demonstrated a low level of metal contamination indicating that reactor corrosion was not significant.

The data for the two runs are summarized in Table 6.

TABLE 6

| | Run 1 | Run 2 |
|---|---|---|
| Solvent Ratio[a] | 4.6 | 4.4 |
| Catalyst | None | None |
| Reaction Temp., °F/°C | 595-605/312.8-318.3 | 600-605/315.6-318.3 |
| Reaction Time, hrs. | 0.5 | 0.33 |
| Times Vented | None | None |
| Filtration Temp.; °F/°C | 258/125.6 | 256/124.4 |
| Washed | Yes | Yes |
| Product Analysis | | |
| Wt.% DM-2,6-NDC | 1.2 | 2.9 |
| Wt.% MM-2,6-NDC | 1.9 | 4.8 |
| Wt.% 2-NA | 0.03 | 0.043 |
| Visual Color | Yellow | Yellow |
| Metals Analyses,[b] ppm | | |
| Na | ND | 1.4 |
| Fe | 2.5 | |
| Al | 1.4 | |
| Cr | 7.8 | 0.8 |

[a] Weight ratio of water to DM-2,6-NDC

[b] Only for metals greater than 1 ppm.

Example 7

The hydrolysis of dimethyl-2,6-naphthalenedicarboxylate was conducted in a 300 ml, stirred, 316 stainless steel reactor fitted with a sample port for venting overhead vapor and a dip-leg sample tube for sampling the bottom, liquid phase of the reaction mixture. A mixture of 100 grams of distilled water and 20 grams of dimethyl-2,6-naphthalenedicarboxylate were used for each run. A hydrolysis catalyst was not added.

The reaction conditions and sample analysis data for three runs are reported in Table 7. Samples designated "1" were taken from the reaction bottom during the reaction. Samples designated as "D" are vapor samples taken at the reaction times indicated. At the end of the reaction, the reactor was rapidly cooled and the remaining liquid was removed by low-temperature distillation. Samples of the resulting liquid are designated "F". Finally, samples designated as "2" were taken from the dried and well-mixed reactor bottoms.

Run 1 was conducted at 329.4°C (625°F). The data from Run 1 indicates that most of the hydrolysis reaction occurs after only 15 minutes. A small amount of 2-NA was formed. No methanol was removed during the run.

Run 2 was conducted at 287.8°C (550°F). Again, high conversion was obtained after only 15 minutes (Sample 2, Run 2). However, in this run some vapor was removed from the reaction mixture (Sample D, Run 2). This sample contained 8.6 percent methanol (by weight) and about 17 grams of total material was removed. After 38 minutes of reaction time the reaction was terminated and the analysis of the product (Sample 2, Run 2) indicated 98.7 percent ester conversion (conversion of ester groups to acid groups). The final vapor sample (Sample F, Run 2) contained only 3 percent methanol compared to 5.4 percent in the sample from the previous run (Sample F, Run 1). This indicates that the removal of methanol during the run provided higher conversion of dimethyl-2,6-naphthalenedicarboxylate compared to Run 1 in Table 7, even at a lower reaction temperature.

Run 3 in Table 7 was conducted at 260°C (500°F) with vapor removal during the run. In this example, 42.7 percent conversion (conversion of ester groups to acid groups) was achieved after 15 minutes (Sample 1, Run 3). After vapor removal (Sample D, Run 3), the reaction was terminated at a total of 43 minutes reaction time. The conversion was 91.8 percent (conversion of ester groups to acid groups). Run 3 in Table 7 demonstrates that, as expected, the reaction rate decreases as the reaction temperature is decreased. However the reaction was still rapid at 260°C (500°F). The Microtrac™ median particle size for the 2,6-NDA produced in Run 3 was 140 microns and the average particle size was 195 microns.

## Table 7

| | Run 1 | | | Run 2 | | | | Run 3 | | |
|---|---|---|---|---|---|---|---|---|---|---|
| Solvent Ratio[a] | 5:1 | | | 5:1 | | | | 5:1 | | |
| Catalyst | None | | | None | | | | None | | |
| Reaction Temp.,°F/°C | 625/329.4 | | | 550/287.8 | | | | 500/260 | | |
| | | | | | | | | | | |
| Sample | 1 | 2 | F | 1 | D | 2 | F | 1 | D | 2 |
| | | | | | | | | | | |
| Sample Location | Bottom | Bottom | Vapor | Bottom | Vapor | Bottom | Vapor | Bottom | Vapor | Bottom |
| Sample Time, min. | 15 | 30 | 30[b] | 15 | 15-22 | 38 | 38[b] | 15 | 16-24 | 43 |
| Sample Weight, g (wet) | | | | | | | | 4.5 | 24.56 | |
| Sample Weight, g (dry) | 0.98 | 15.9 | - | 0.6 | 0.05 | 16.4 | | 0.937 | 0.076 | 16.01 |
| | | | | | | | | | | |
| **Product Composition** | | | | | | | | | | |
| | | | | | | | | | | |
| **Vapor, wt%:** | | | | | | | | | | |
| MeOH | | | 5.4 | | 8.61 | | 3 | | 7.23 | |
| DME | | | 0.35 | | 0.22 | | 0 | | 0.054 | |
| | | | | | | | | | | |
| **Reactor Bottom, Wt.%** | | | | | | | | | | |
| **(after drying)** | | | | | | | | | | |
| 2,6-NDA | 80.03 | 84.35 | | 67.3 | | 94.78 | | 17.28 | | 83.44 |
| DM-2,6-NDC | 0.55 | 0.33 | | 1.72 | | 0.14 | | 33.55 | | 2.27 |
| MN-2,6-NDC | 15.60 | 12.30 | | 25.21 | | 2.48 | | 40.30 | | 12.77 |
| 2-NA | 0.35 | 0.70 | | 0.00 | | 0.00 | | 0.00 | | 0.00 |
| Others | 0.07 | 0.17 | | 0.15 | | 0.06 | | 0.20 | | 0.11 |
| | | | | | | | | | | |
| Totals | 96.60 | 97.85 | | 94.81 | | 97.46 | | 91.33 | | 98.59 |
| | | | | | | | | | | |
| **Reactor Bottom, mole %** | | | | | | | | | | |
| 2,6-NDA | 84.1 | 87.7 | | 72.9 | | 97.5 | | 20.4 | | 85.6 |
| DM-2,6-NDC | 0.5 | 0.3 | | 1.6 | | 0.1 | | 35.0 | | 2.1 |
| MM-2,6-NDC | 15.4 | 12.0 | | 25.5 | | 2.4 | | 44.6 | | 12.3 |
| | | | | | | | | | | |
| Total Mole % Ester Groups | 8.2 | 6.3 | | 14.4 | | 1.3 | | 57.3 | | 8.2 |
| Total Mole % Acid Groups | 91.8 | 93.7 | | 85.6 | | 98.7 | | 42.7 | | 91.8 |

[a]  Weight ratio of water to DM-2,6-NDC charged to reactor.
[b]  At end of reaction.

EP 0 572 628 B2

Example 8

This example reports the crystal morphology for 2,6-naphthalenedicarboxylic acid produced by the process of this invention as well as by a low-temperature hydrolysis process such as that reported in Example 1. The morphology was determined by scanning electron microscopy analysis.

| Hydrolysis Process | 2,6-NDA Particle Morphology |
|---|---|
| Low-temperature hydrolysis catalyzed by p-toluene sulfonic acid. 5:1 weight ratio of water to DM-2,6-NDC. Reaction temperature of 198.9-204.4°C (390-400°F). | Particles up to about 6 x $10^{-5}$ m (60 microns) at low magnification. At high magnification, particles appear as highly porous agglomerates of small crystals wherein the small crystals have a length of about $10^{-6}$ m (1 micron) and a width of a few tenths of $10^{-6}$ m (a micron). |
| High-temperature hydrolysis. No catalyst. 4:1 weight ratio of water to DM-2,6-NDC. Reaction temperature 308.3°C (587°F). 2,6-NDA prepared by Example 3, Run 1. | Particles are well-formed, substantially non-porous crystals with a median particle size of greater than $10^{-4}$ m (100 microns) as measured by the Microtrac™ particle analyzer. |
| High-temperature hydrolysis. No catalyst. 4:1 weight ratio of water to DM-2,6-NDC. Reaction temperature of 280.6°C (537°F). 2,6-NDA prepared by Example 3, Run 3. | Particles typically greater than $10^{-4}$ m (100 microns) in length, consisting of agglomerates of smaller crystals having a length of about 2-5 x $10^{-5}$ m (20-50 microns) and a width of about 5-10 x $10^{-5}$ m (5-10 microns). |

Example 9

This example reports the results of a qualitative analyses conducted to determine the amount of ethylene glycol that is required to form pumpable slurries of 2,6-naphthalenedicarboxylic acid in ethylene glycol.
Reported below are the minimum grams of ethylene glycol per gram of 2,6-naphthalenedicarboxylic acid that are required to form a "paste" and a "slurry". "Paste" is defined as a mixture with all the crystals wetted. "Slurry" is defined as a mixture that has a smooth, creamy consistency

| Hydrolysis Process | Paste | Slurry |
|---|---|---|
| 2,6-NDA produced by low-temperature process as reported in Example 1. | 0.802 | 0.966 |
| Process of this invention as reported in Example 3, Run 1. | 0.513 | 0.666 |

Although these data are qualitative, they demonstrate that the 2,6-naphthalenedicarboxylic acid produced by the low-temperature process requires approximately 50% more ethylene glycol to produce a slurry compared to the 2,6-NDA produced at 310°C (590°F).

Example 10

Table 8 reports the Brookfield viscosities of mixtures of ethylene glycol and 2,6-naphthalenedicarboxylic acid. These data demonstrate that the 2,6-naphthalenedicarboxylic acid prepared in Example 6 according to the process of this invention provides for a substantially less viscous mixture of 2,6-naphthalenedicarboxylic acid and ethylene glycol compared to the 2,6-naphthalenedicarboxylic acid prepared by a low-temperature process as exemplified by Example 1.

TABLE 8

| Mole Ratio Ethylene Glycol: 2.6-NDA | Brookfield Viscosity[a] | |
| --- | --- | --- |
| | 2.6-NDA (Example 6) | 2.6-NDA Low-Temp. Process[b] |
| 2.0 | 12,373 | |
| 2.5 | 2,480 | |
| 3.0 | 800 | |
| 3.5 | 373 | 20,160 |
| 4.0 | | 10,293 |
| 4.5 | | 6,240 |
| 5.0 | | 4,320 |
| 5.5 | | 3,253 |
| 6.0 | | 2,400 |
| 6.5 | | 1,760 |
| 7.0 | | 1,280 |
| 7.5 | | 1,120 |
| 8.0 | | 960 |
| 8.5 | | 800 |

[a] Viscosities in centipoise measured using a #4 spindle at room temperature (ca 70°F) and at 50 rpm.

[b] Prepared by process equivalent to that reported in Example 1 using a reaction temperature of 193.3-204.4°C (380-400°F) and a weight ratio of water to DM-2,6-NDC of 5:1.

Example 11

Solubility data for 2;6-naphthalenedicarboxylic acid in distilled water is listed below. These data can be used to calculate the percent 2,6-NDA solubilized in water at the listed reaction temperature.

| Temperature (C/°F) | Solubility (grams 2.6-NDA/100 g $H_2O$) |
| --- | --- |
| 160/320 | 0.041 |
| 200/392 | 0.22 |
| 240/464 | 1.19 |
| 280/536 | 6.07 |
| 320/608 | 33.2 |

A plot of this data as the Ln(grams 2,6-NDA/100 grams water) vs. temperature is linear and, therefore, provides for other solubility values by interpolation or extrapolation.

Example 12

A sample of crude 2,6-naphthalenedicarboxylic acid having the composition reported in Table 9 was dissolved in 5 parts by weight of distilled water at 332.2°C (630°F) and held there for 10 minutes. For each of the three runs reported in Table 9, the solution of 2,6-naphthalenedicarboxylic acid was cooled, at the cooling rates shown, to 204.4°C (400°F). At 204.4°C (400°F) the crystallized 2,6-NDA was separated from the mother liquor.

These data demonstrate that the particle size of the resulting 2,6-naphthalenedicarboxylic acid is a function of the cooling rate. These data also demonstrate the preparation of 2,6-naphthalenedicarboxylic acid having an average (mean) particle size of $1.33-2.07 \times 10^{-4}$ m (133-207 microns).

TABLE 9

| Composition[a] | Crystallized 2,6-NDA | | | |
| --- | --- | --- | --- | --- |
| | Feed | Run 1 | Run 2 | Run 3 |
| 2,6-NDA | 93.0 | 93.0 | 93.0 | 93.0 |
| 2,7-NDA | 0.01 | 0.00 | 0.00 | 0.00 |
| Br-2,6-NDA | 0.55 | 0.24 | 0.16 | 0.06 |

TABLE 9   (continued)

| Compositiona | Feed | Crystallized 2,6-NDA | | |
|---|---|---|---|---|
| | | Run 1 | Run 2 | Run 3 |
| 2-FNA | 0.62 | 0.59 | 0.55 | 0.53 |
| 2-NA | 0.00 | 0.12 | 0.10 | 0.18 |
| TMA | 0.36 | 0.11 | 0.07 | 0.07 |
| Others | 0.93 | 2.18 | 1.96 | 2.94 |
| Total | 95.72 | 96.73 | 95.85 | 97.19 |
| Cooling Rate, °F/min./°C/min | | 125/69.4 | 16/8.9 | 6.5/3.6 |
| Median Particle Size x $10^{-6}$ m (Microns) | | 50 | 118 | 169 |
| Mean Particle Size (Microns) x $10^{-6}$ m | | 54 | 133 | 207 |
| %<11 microns (x $10^{-6}$ m) | | 2.2 | 0 | 0 |
| % Solids recovered | | 95.4 | 97.6 | 98.2 |

**Claims**

1. A process for preparing purified 2,6-naphthalenedicarboxylic acid comprising:

     a) hydrolyzing, in the absence or substantial absence of at least one of (i) a hydrolysis catalyst, and (ii) a monocarboxylic acid cosolvent, a dialkyl-2,6-naphthalenedicarboxylate with water at a reaction temperature of at least 232.2°C (450°F) under liquid phase conditions and for a time sufficient to convert a major portion of the dialkyl-2,6-naphthalenedicarboxylate to 2,6-naphthalenedicarboxylic acid thereby forming a reaction product mixture, the amount of water present being sufficient to solubilize, at the reaction temperature, at least 10 weight percent of the 2,6-naphthalenedicarboxylic acid formed; and
     b) recovering 2,6-naphthalenedicarboxylic acid from the reaction product mixture.

2. A process according to Claim 1 wherein the amount of water present is an amount sufficient to solubilize at least 50 weight percent of the 2,6-naphthalenedicarboxylic acid present in the reaction product mixture.

3. A process according to Claim 1 wherein the amount of water present in an amount sufficient to solubilize substantially all of the 2,6-naphthalenedicarboxylic acid present in the reaction product mixture.

4. A process according to any of Claims 1 to 3 wherein the reaction temperature is at least 298.9°C (570°F).

5. A process according to any of Claims 1 to 4 wherein the dialkyl-2,6-naphthalenedicarboxylate, is dimethyl 2,6-naphthalenedicarboxylate.

6. Substantially non-porous 2,6-naphthalenedicarboxylic acid crystals having an average particle size of at least $10^{-4}$m (100 microns).

7. Substantially non-porous 2,6-napthalenedicarboxylic acid crystals having an average particle size of at least 1.25 x $10^{-4}$m (125 microns).

8. Substantially non-porous 2,6-naphthalenedicarboxylic acid crystals having an average particle size of least 1.75 x $10^{-4}$m (175 microns).

9. Substantially non-porous 2,6-naphthalenedicarboxylic acid crystals having an average particle size of at least 1.95 x $10^{-4}$m (195 microns)

10. A composition comprising 2,6-naphthalenedicarboxylic acid and a glycol wherein the 2,6-naphthalenedicarboxylic acid has an average particle size of at least $10^{-4}$ m (100 microns).

11. A composition consisting essentially of 2,6-naphthalenedicarboxylic acid and ethylene glycol having a Brookfield viscosity of no more than 1 Pas (1000 centipoise) and a mole ratio of ethylene glycol to 2,6-naphthalenedicarboxylic acid of no more than 4:1, respectively.

12. A composition according to Claim 10 wherein the Brookfield viscosity is no more than 3 Pa s (3000 centipoise) and the mole ratio of ethylene glycol to 2,6-naphthalenedicarboxylic acid is no more than 3:5:1, respectively.

**Patentansprüche**

1. Verfahren zur Herstellung gereinigter 2,6-Naphthalindicarbonsäure, das umfaßt:

a) das Hydrolysieren - ohne oder im wesentlichen ohne wenigstens eines der Materialien: (i) ein Hydrolysierungskatalystor und (ii) ein Monocarbonsäure-Colösungsmittel eines Dialkyl-2,6-naphthalindicarboxylats mit Wasser bei einer Reaktionstemperatur von mindestens 232,2° C (450° F) unter Flüssigphasenbedingungen und für eine ausreichende Zeitspanne, um einen Hauptteil des Dialkyl-2,6-naphthalindicarboxylats in 2,6-Naphthalindicarbonsäure umzuwandeln und dabei eine Reaktionsproduktmischung zu bilden, wobei die Menge an vorhandenem Wasser ausreicht, um bei der Reaktionstemperatur wenigstens 10 Gew.-% der gebildeten 2,6-Naphthalindicarbonsäure löslich zu machen und
b) Gewinnung von 2,6-Naphthalindicarbonsäure aus der Reaktionsmischung.

2. Verfahren nach Anspruch 1, wobei die Menge an vorhandenem Wasser ausreichend ist, um etwa 50 Gew.-% der in der Reaktionsproduktmischung vorhandenen 2,6-Naphthalindicarbonsäure löslich zu machen.

3. Verfahren nach Anspruch 1, wobei die Menge an vorhandenem Wasser ausreichend ist, um im wesentlichen die gesamte, in der Reaktionsproduktmischung vorhandene 2,6-Naphthalindicarbonsäure löslich zu machen.

4. Verfahren nach einem der Ansprüche 1 bis 3, wobei die Reaktionstemperatur wenigstens 298,9° C (570° F) beträgt.

5. Verfahren nach einem der Ansprüche 1 bis 4, wobei das Dialkyl-2,6-naphthalindicarboxylat Dimethyl-2,6-naphthalindicarboxylat ist.

6. Im wesentlichen nicht-poröse 2,6-Naphthalindicarbonsäure-Kristalle mit einer durchschnittlichen Teilchengröße von wenigstens $10^{-4}$ m (100 Mikron).

7. Im wesentlichen nicht-poröse 2,6-Naphthalindicarbonsäure-Kristalle mit einer durchschnittlichen Teilchengröße von wenigstens $1,25 \times 10^{-4}$ m (100 Mikron).

8. Im wesentlichen nicht-poröse 2,6-Naphthalindicarbonsäure-Kristalle mit einer durchschnittlichen Teilchengröße von wenigstens $1,75 \times 10^{-4}$ m (100 Mikron).

9. Im wesentlichen nicht-poröse 2,6-Naphthalindicarbonsäure-Kristalle mit einer durchschnittlichen Teilchengröße von wenigstens $1,95 \times 10^{-4}$ m (100 Mikron).

10. Zusammensetzung, die 2,6-Naphthalindicarbonsäure und ein Glykol umfaßt, wobei die 2,6-Naphthalindicarbonsäure eine durchschnittliche Teilchengröße von wenigstens $10^{-4}$ m (100 Mikron) beträgt.

11. Zusammensetzung, die im wesentlichen aus 2,6-Naphthalindicarbonsäure und Ethylenglykol besteht und eine Brookfield-Viskosität von nicht mehr als 1 Pa s (1000 Centipoise) bzw. ein Molverhältnis von Ethylenglykol zu 2,6-Naphthalindicarbonsäure von nicht mehr als 4:1 aufweist.

12. Zusammensetzung nach Anspruch 10, wobei die Brookfield-Viskosität nicht mehr als 3 Pa s (3000 Centipoise) bzw. das Molverhältnis von Ethylenglykol zu 2,6-Naphthalindicarbonsäure nicht mehr als 3,5:1 aufweist.

**Revendications**

1. Procédé pour préparer un acide 2,6-naphtalènedicarboxylique purifié, qui comprend les étapes consistant :

 a) à hydrolyser, en l'absence ou en l'absence presque complète d'au moins (i) un catalyseur d'hydrolyse ou bien (ii) un co-solvant de type acide monocarboxylique, un 2,6-naphtalènedicarboxylate de dialkyle avec de l'eau, à une température de réaction d'au moins 232,2°C (450°F) dans les conditions d'une phase liquide et pendant un laps de temps suffisant pour convertir une partie principale du 2,6-naphtalènedicarboxylate de dialkyle en acide 2,6-naphtalènedicarboxylique, de façon à former un mélange de produits de réaction, la quantité présente d'eau étant suffisante pour solubiliser, à la température de la réaction, au moins 10 % en poids de l'acide 2,6-naphtalènedicarboxylique formé ; et
 b) à récupérer du mélange de produits de réaction l'acide 2,6-naphtalènedicarboxylique.

2. Procédé selon la revendication 1, dans lequel la quantité d'eau présente est une quantité suffisante pour solubiliser au moins 50 % en poids de l'acide 2,6-naphtalènedicarboxylique présent dans le mélange de produits de réaction.

3. Procédé selon la revendication 1, dans lequel la quantité d'eau présente est suffisante pour solubiliser la presque totalité de l'acide 2,6-naphtalènedicarboxylique présent dans le mélange de produits de réaction.

4. Procédé selon l'une quelconque des revendications 1 à 3, dans lequel la température de réaction est d'au moins 298,9°C (570°F).

5. Procédé selon l'une quelconque des revendications 1 à 4, dans lequel le 2,6-naphtalènedicarboxylate de dialkyle est le 2,6-naphtalènedicarboxylate de diméthyle.

6. Cristaux d'acide 2,6-naphtalènedicarboxylique essentiellement non poreux ayant une granulométrie moyenne d'au moins $10^{-4}$ m (100 micromètres).

7. Cristaux d'acide 2,6-naphtalènedicarboxylique essentiellement non poreux ayant une granulométrie moyenne d'au moins $1,25.10^{-4}$ m (125 micromètres).

8. Cristaux d'acide 2,6-naphtalènedicarboxylique essentiellement non poreux ayant une granulométrie moyenne d'au moins $1,75.10^{-4}$ m (175 micromètres).

9. Cristaux d'acide 2,6-naphtalènedicarboxylique essentiellement non poreux ayant une granulométrie moyenne d'au moins $1,95.10^{-4}$ m (195 micromètres).

10. Composition comprenant de l'acide 2,6-naphtalènedicarboxylique et un glycol, dans laquelle l'acide 2,6-naphtalènedicarboxylique à une granulométrie moyenne d'au moins $10^{-4}$ m (100 micromètres).

11. Composition constituée essentiellement d'acide 2,6-naphtalènedicarboxylique et d'éthylèneglycol, ayant une viscosité Brookfield non supérieure à 1 Pa.s (1000 centipoises) et un rapport en moles de l'éthylèneglycol à l'acide 2,6-naphtalènedicarboxylique non supérieur à 4:1, respectivement.

12. Composition selon la revendication 10, dans laquelle la viscosité Brookfield n'est pas supérieure à 3 Pa.s (3000 centipoises), et le rapport en moles de l'éthylèneglycol à l'acide 2,6-naphtalènedicarboxylique n'est pas supérieur à 3,5:1, respectivement.

FIG. 1